# EUROPEAN PATENT SPECIFICATION

(11) **EP 3 345 004 B1**
(45) Date of publication and mention of the grant of the patent: **22.07.2020**
(21) Application number: 16762849.4
(22) Date of filing: 02.09.2016
(51) Int. Cl.: G01N 33/92

(54) **DIAGNOSTIC METHODS AND KITS**
DIAGNOSEVERFAHREN UND KITS
MÉTHODES DIAGNOSTIQUES ET KITS

(30) Priority: 02.09.2015 GB 201515573; 16.09.2015 GB 201516441; 13.05.2016 GB 201608459
(43) Date of publication of application: 11.07.2018
(73) Proprietor: Cholestenix Limited, Swansea Enterprise Park Swansea SA7 9FS (GB)
(72) Inventor: GRIFFITHS, William, Swansea West Glamorgan SA2 8PP (GB); WANG, Yuqin, Swansea West Glamorgan SA2 8PP (GB); ABDEL-KHALIK, Jonas, Swansea West Glamorgan SA2 8PP (GB)
(74) Representative: CSY London
(86) International application number: PCT/GB2016/052710
(87) International publication number: WO 2017/037465

(56) References cited:
- WO-A1-2008/137767
- WO-A2-2014/132052
- I. BJORKHEM ET AL: "On the formation of 7-ketocholesterol from 7-dehydrocholesterol in patients with CTX and SLO", JOURNAL OF LIPID RESEARCH, vol. 55, no. 6, 1 June 2014 (2014-06-01), pages 1165-1172, XP055314213, US ISSN: 0022-2275, DOI: 10.1194/jlr.P048603
- Gunvor Alvelius ET AL: "Identification of unusual 7-oxygenated bile acid sulfates in a patient with Niemann-Pick disease, type C 1", Journal of Lipid Research Volume, vol. 42 2001, page 1571, XP055314211, Retrieved from the Internet: URL:http://www.jlr.org/content/42/10/1571. full.pdf
- VANIER MARIE T: "Complex lipid trafficking in Niemann-Pick disease type C", JOURNAL OF INHERITED METABOLIC DISEASE, KLUWER, DORDRECHT, NL, vol. 38, no. 1, 26 November 2014 (2014-11-26), pages 187-199, XP035416971, ISSN: 0141-8955, DOI: 10.1007/S10545-014-9794-4 [retrieved on 2014-11-26]

## Description

### Background of the Invention

Oxysterols are usually thought of as oxidised forms of cholesterol. However, they can also be formed from cholesterol precursors and the resulting products can have biological activity. 7-Dehydrocholesterol (7-DHC) is an immediate precursor of cholesterol; it is converted to cholesterol by the enzyme 7-dehydrocholesterol reductase (DHCR7). Mutations in DHCR7 leading to reduced enzyme activity result in accumulation of 7-DHC and the disease Smith-Lemli-Opitz syndrome (SLOS). SLOS may present as a malformation syndrome with a distinctive cognitive phenotype. Whether the disease is a consequence of reduced cholesterol synthesis or accumulation of 7-DHC or its metabolites is unknown. Furthermore, little is known of how 7-DHC is metabolised other than it can be isomerised to 8-DHC.

Bile acids are a large family of steroids possessing a carboxyl group on the side-chain. They are largely synthesised in the liver, but also extrahepatically and there is compelling evidence for their biosynthesis in the brain. Bile acids are synthesised predominantly via two pathways. The dominating pathway in man is the neutral or normal pathway which starts with 7α-hydroxylation of cholesterol by the hepatic cytochrome P450 (CYP) 7A1 enzyme. The second pathway, known as the acidic pathway, starts with 25R(26)-hydroxylation of cholesterol by CYP27A1 to give (25R)26-hydroxycholesterol either in the liver or extrahepatically.

The systematic numbering system according to IUPAC rules is used herein with respect to the (25R)26-hydroxylation of cholesterol. However, much of the literature describes the resulting product as 27-hydroxycholesterol.

Other minor pathways begin with 25-hydroxylation of cholesterol by cholesterol 25-hydroxylase (CH25H) in activated macrophages or with 24S-hydroxylation of cholesterol by cholesterol 24S-hydroxylase (CYP46A1) in brain. Many of the subsequent enzymes converting hydroxycholesterols to bile acids are operative in multiple pathways allowing metabolite crossing between pathways.

The major bile acids in man are cholic acid, chenodeoxycholic acid, ursodeoxycholic, deoxycholic acid and lithocholic acid. The latter two are derived from the former two by 7α-dehyroxylation. They are secreted in bile as glycine or taurine conjugates, or in the case of lithocholic acid as a 3-sulphate. Bile acids function in the intestine to aid absorption of lipids, and are recycled to the liver via the enterohepatic system. As well as functioning as detergents in the intestine bile acids are also signalling molecules, regulating their own synthesis via interaction with the farnasoid X receptor, while intermediates in their biosynthetic pathways from cholesterol are ligands to nuclear receptors e.g. liver X receptors (LXRs), pregnane X receptor (PXR), vitamin D receptor (VDR), constitutive androstane receptor (CAR) and to G-protein coupled receptors (GPCRs) e.g. EBI2 and TGR5.

Interestingly, there is increasing evidence for an involvement of bile acid biosynthesis pathways in the nervous system. Almost all of the acidic pathway intermediates from cholesterol to bile acids can be found in brain or cerebrospinal fluid (CSF), and many of these intermediates can cross the blood brain barrier providing traffic in and out of the CNS. Cholic acid has been identified in brain and shown to act as a ligand to LXRs regulating the neurogenesis of red nucleus neurons, while the C₂₇ bile acid 3β,7α-dihydroxycholest-5-en-(25R)26-oic acid (3β,7α-diHCA) has been shown to regulate the survival of motor neurons, again through interaction with LXRs.

It is now accepted that bile acid biosynthesis not only provides detergent molecules essential in the intestine, but also numerous signalling molecules important in a diverse array of biological processes. Unsurprisingly, deficiency in enzymes of the bile acid biosynthesis pathways lead to disease [Setchell JPGN 2006], however, as a consequence of the redundancy provided by multiple pathways, often not to a total elimination of bile acid formation e.g. cerebrotendinous xanthomatosis (CTX) where there is a deficiency of CYP27A1 but cholic acid formation is maintained [Bjorkhem BBRC 2010].

Likewise defects in cholesterol biosynthesis result in clinical disorders [Shackleton Steroids 2012], however, it is unknown if there is sufficient metabolic redundancy for cholesterol to be bypassed and bile acid biosynthesis still maintained by yet another metabolic pathway.

In I. Bjorkhem ET AL, Journal of Lipid Research, vol. 55, no.6, 1 June 2014, pages 1165-1172, it is shown that patients with SLO have elevated levels of 7-ketocholesterol and hence, this is considered as a diagnostic marker of SLOS.

WO2014/132052 teaches 3β, 7α-di HCA for use in the treatment of neurodegenerative conditions.

WO2008/137767 mentions the use of tandem mass spectrometry for detecting and/or screening for conditions associated with altered sterols.

The applicants have found that bile acids can be biosynthesised from 7-dehydrocholesterol (7-DHC), an immediate precursor of cholesterol, in patients with conditions caused by defective cholesterol biosynthesis such as the disorder Smith-Lemli-Opitz syndrome (SLOS), where the enzyme 7-dehydrocholesterol reductase (DCHCR7) is deficient and 7-DHC is abundant in tissues and plasma.

The SLOS phenotype is very broad; severely affected cases often die *in utero* or soon after birth, whereas mild cases show only minor physical abnormalities, together with learning and behavioural problems. Limb abnormalities are common in SLOS, and in addition to physical malformations SLOS patients have impaired cognitive function although normal intelligence is also possible. Dietary cholesterol supplementation and treatment with statins is standard. Although the primary enzymatic defect in SLOS is well defined, its pathophysiology is not, and it is unlikely that one mechanism explains the myriad of symptoms.

The applicants have found a range of compounds which appear to be associated with SLOS or which are found at elevated levels in SLOS patients. Some of these compounds are indicative of the presence of a new biosynthetic pathway associated with conditions such as SLOS. This leads to the provision of novel methods of diagnosis or prognosis of these conditions. Furthermore, certain intermediates in the pathway are ligands to Smoothened, an oncoprotein activated during Hedgehog signalling required for embryonic patterning and regeneration of postembryonic tissue. Accordingly, the compounds may be formulated as a pharmaceutical preparation comprising a pharmaceutically acceptable excipient. Compounds of the invention and/or preparations comprising them may be administered to a patient to treat conditions involving unwanted cell proliferation, e.g., cancer and/or tumors (such as medulloblastoma, basal cell carcinoma, etc.), non-malignant hyperproliferative disorders, etc. The compounds can also be used to regulate the growth and differentiation of normal tissues. In certain embodiments, such compounds or preparations are administered systemically and/or locally, e.g., topically.

### Summary of the Invention

According to the present invention there is provided a method for diagnosing Smith-Lemli-Opitz syndrome (SLOS) comprising detecting levels of the compound of formula (I) or a derivative thereof in a urine sample from a subject suspected of or suffering from SLOS, or from a urine sample from an expectant mother 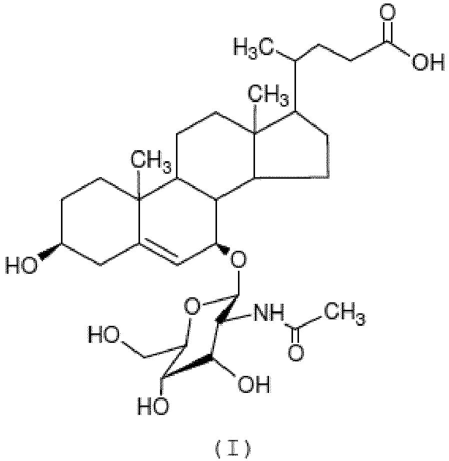 comparing these with the levels found in healthy subject
which are higher than those found in a sample from a subject not suffering from SLOS.

### Detailed Description and Preferred Embodiments.

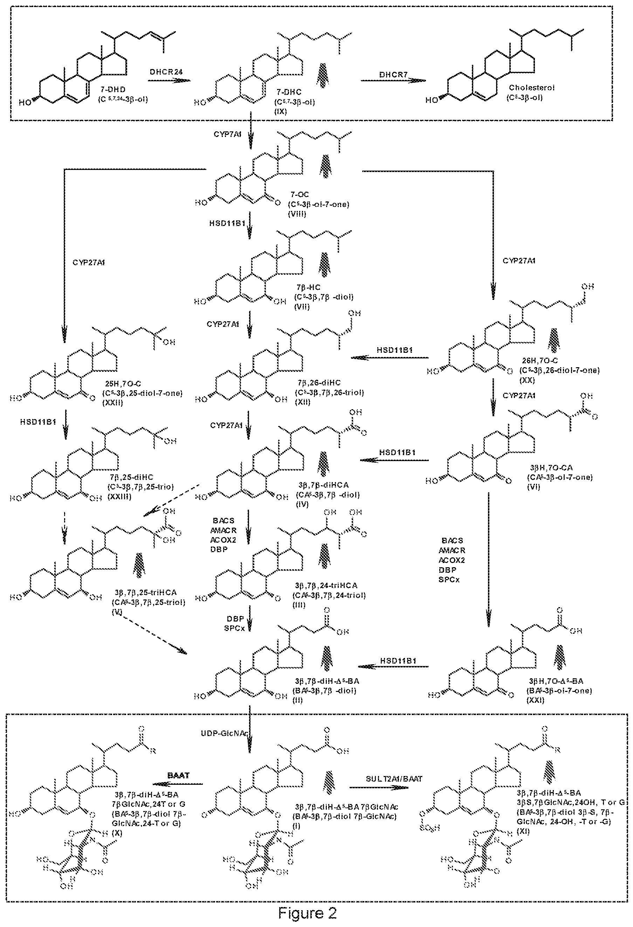

The applicants have found that the compound of formula (I) is a product in a novel biosynthetic pathway (as shown in Figure 2 hereinafter) found in patients suffering from conditions caused by defective cholesterol biosynthesis, in particular Smith-Lemli-Opitz syndrome (SLOS). As a result, this product or precursors as described above may be detected at elevated levels in biological samples including blood, plasma, serum, cerebrospinal fluid (CSF) or urine samples.

Precursors of the compound of formula (I) in the biosynthetic pathway are compounds of formulae (II)-(VII) and (XX)-(XXIII) as defined above together with compounds of formulae (VIII) and (IX)

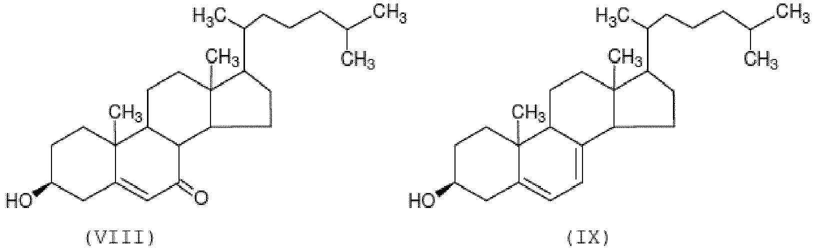

Although it has previously been reported that compounds of formulae (VIII) and (IX) above, were present in elevated levels in SLOS patients, it had not previously been understood that they were part of the pathway of Figure 2. Furthermore, they are present in samples from healthy patients in widely variable amounts, which can make it difficult to identify elevated levels which are specifically indicative of conditions such as SLOS.

Compounds of formula (II)-(VII), and (XX)-(XXIII) and in particular compounds of formula (II)- (VI) and (XX) are much better diagnostic markers than compounds of formula (VIII) or (IX) as they are present at low or trace amounts in healthy individuals, and so elevation as a result of a condition such as SLOS, is more easily determined.

A further precursor of formula (XII) in the pathway 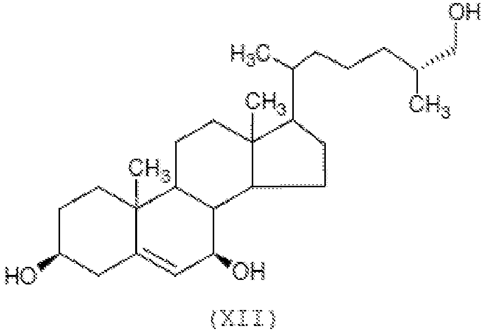 is found only in low levels which are difficult to measure accurately, making it less useful as a diagnostic moiety.

Suitably, precursors of formulae (II)-(VII), and (XX)-(XXIII) are detected at elevated levels in blood, serum, plasma or CSF samples from a subject to provide a diagnosis of SLOS in said subject, or to monitor the progress of the disease in subjects known to be suffering from SLOS.

It has been found however that compounds of formula (I) and derivatives thereof are markers that may be found additionally in urine. Urinary markers are preferred for diagnostic purposes as sample retrieval is easy and less invasive for the subject. In addition, however, a urinary marker may be used in pre-natal diagnosis. The presence of elevated levels of the compound of formula (I) or derivatives thereof in a maternal urine sample would be indicative of the presence of a condition such as SLOS in the foetus.

Thus, in a particular embodiment, the invention provides a method for diagnosing SLOS which comprises detecting levels of the compound of formula (I) or a derivative thereof, in a urine sample from a subject suspected of or suffering from SLOS or from a urine sample from an expectant mother and comparing these with the levels found in healthy subject.

Suitable derivatives of Compounds of formula (I) include compounds of formula (X) and (XI) 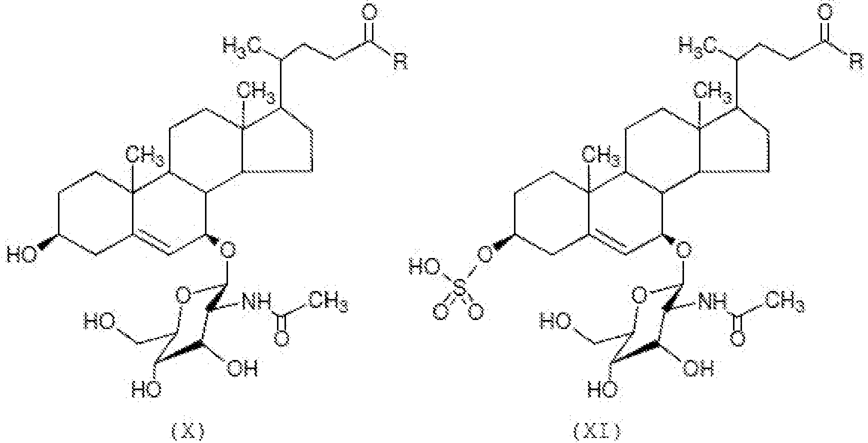 wherein R is a hydroxyl, glycine (-NHCH₂CO₂H) or taurine (-NHCH₂CH₂SO₃H) group.

In particular in the compound of formula (X), R is a glycine or taurine group, and in the compound of formula (XI), R is a hydroxyl group.

Compounds of formula (I) as well as compounds of formula (X) and (XI) have previously been detected in the urine of a patient suffering from Niemann Pick disease, an inherited lipid trafficking disorder, (G. Alvelius et al. (2001) The Journal of Lipid Research, 42, 1571-1577, October 2001; The Journal of Lipid Research, 42, 1571-1577) but they have not previously been associated with conditions caused by defective cholesterol biosynthesis such as SLOS. Niemann Pick disease is quite a distinct ailment, presenting different symptoms from those present in, for example, SLOS and thus is unlikely to be confused in patients. However, confirmatory tests for SLOS, for example for detecting a second compound which acts as a diagnostic marker for conditions such as SLOS as described below may be carried out in addition to the method of the invention in order to confirm a diagnosis of a condition caused by defective cholesterol biosynthesis, such as SLOS.

As used herein, the expression 'elevated level' refers to levels of a compound which is higher, in particular at least 1.5 times, for example at least double, the level of the compound found in a sample taken from healthy subjects, who are not suffering from the condition such as SLOS (See Table 1 hereinafter). These levels may be determined empirically or by comparison with a previously determined standard level. Typically, these compounds are found at low levels in samples taken from healthy individuals, for example at levels of 1 ng/ml or less. Thus, levels of compounds of formula II, IV, VI and VII excess of 5ng/ml, or in excess of 10, 20 or 30 ng/ml in a sample may be indicative of the presence of the condition.

In a particular embodiment, the method of the invention further comprises detecting a further compound or diagnostic marker which is characteristic of a condition caused by defective cholesterol biosynthesis such as SLOS. Such tests would provide further reassurance of the veracity of the result. In addition to the compounds described above, the applicants explored patterns of other oxysterols found in plasma of patients with SLOS exploiting Girard derivatisation and LC-MS via a technology designated 'enzyme assisted derivatisation for sterol analysis' or EADSA.

They have additionally identified, for the first time, the 8-DHC metabolites 24-hydroxy-8-DHC (24-OH-8-DHC), 25-OH-8-DHC and 26-OH-8-DHC as well as elevated levels of the known 7-DHC metabolites 4-OH-7-DHC, 7-oxocholesterol (Compound (VIII) above), 7α,8α-epoxycholesterol and 3β,5α-dihydroxycholest-7-en-6-one.

Certain of these compounds may also provide means of diagnosis of conditions such as SLOS, in particular as a confirmatory test, to provide a clear differentiation of distinction from this and Niemann Pick disorder, and this forms a further aspect of the invention.

In particular, in a particular embodiment, the method of the invention further provides the detection of elevated levels of 8-dehydocholesterol (8-DHC) of formula (XIII) 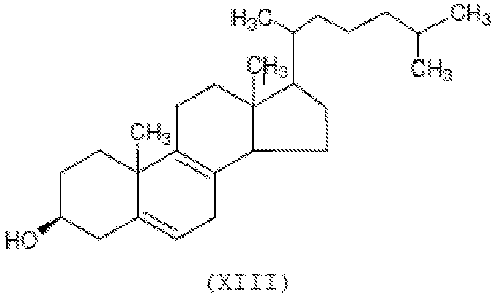 or a metabolite thereof, selected from 24-hydroxy-8-DHC (24-OH-8-DHC) of formula (XIV), 25-OH-8-DHC of formula (XV) and 26-OH-8-DHC of formula (XVI), 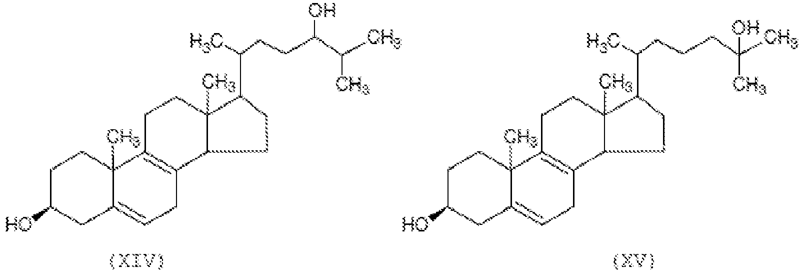 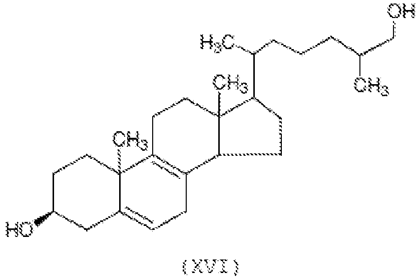 and in particular, compounds of formula (XIV) and (XV) above.

Alternatively or additionally, the further step may comprise the detection of levels of 7-DHC of formula (IX) above or 7-DHC metabolites such as 4-OH-7-DHC of formula (XVII), 7-oxocholesterol (Compound VIII), 7α,8α-epoxycholesterol (XVIII) and 3β,5α-dihydroxycholest-7-en-6-one (Compound XIX) which are higher than those found in a sample from a subject not suffering from said condition

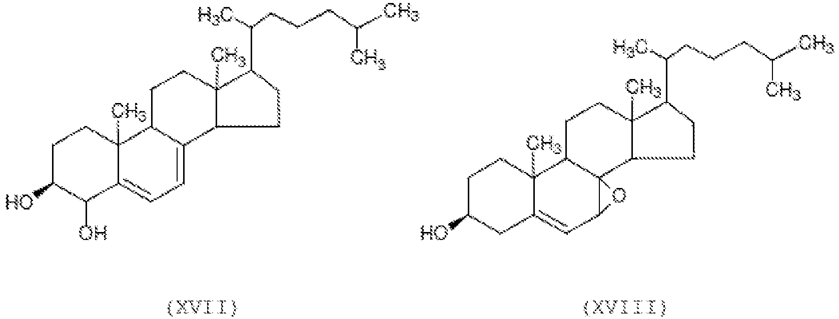

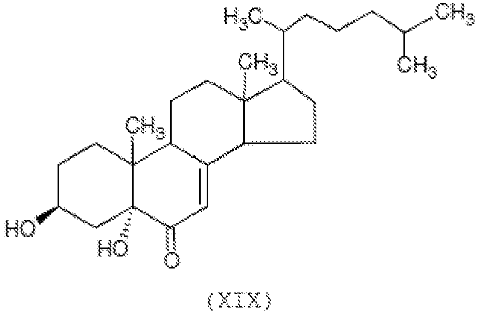

Detection of the compounds of formula (I)-(XXIII) may be carried out using conventional methods, in particular using liquid chromatography combined with mass spectrometry, particularly following derivatisation such as Girard derivatisation with reagents such are Girard P to facilitate detection of specific forms. These compounds may be identified and quantified using methods known in the art.

Bile acid biosynthesis normally starts from cholesterol, however, CYP7A1 can also use 7-DHC as a substrate giving 7-oxocholesterol (VIII) which can be reduced by HSD11B1 to 7β-hydroxycholesterol (VII) opening a new route to bile acid biosynthesis (Figure 2). The elevated levels of these sterols in plasma of SLOS patients and also those of 3β,7β -dihydroxycholest-5-en-26-oic acid (IV) and 3β,7β-dihydroxychol-5-en-24-oic acid (II) define a new and unexpected pathway for bile acid biosynthesis in SLOS patients.

Further evidence for this pathway was provided by the presumptive identification of 3β,7β,24-trihydroxycholest-5-en-26-oic acid (Compound III), a necessary intermediate as the CoA thioester in peroxisomal side-chain shortening of 3β,7β-dihydroxycholest-5-en-26-oic acid (IV) to 3β,7β-dihydroxychol-5-en-24-oic acid (II). A second branch to the pathway is defined by the identification of elevated amounts of 3β,26-Dihydroxycholest-5-en-7-one (26-Hydroxy-7-oxocholesterol) (Compound XX) and 3β-Hydroxy-7-oxochol-5-en-24-oic acid (Compound XXI) in the plasma of SLOS patients, and also the identification of elevated amounts of 3β-hydroxy-7-oxocholest-5-en-26-oic acid (VI) in plasma of patients with a high 7-DHC to cholesterol ratio, which can also presumably act as a substrate for HSD11B1. A third branch to the pathway proceeds through 3β,25-dihydroxycholest-5-en-7-one (Compound XXII), 7β,25-dihydroxycholesterol (Compound XXIII) and 3β,7β,25-trihydroxycholest-5-en-26-oic acid (Compound V), although it is not known whether CYP27A1 is responsible for the oxidation of the terminal carbon to the carboxylic acid and how the resulting triol undergoes side-chain shortening. The 7β-hydroxy group in bile acids is known to become conjugated with (N-Acetylglucosamine) GlcNAC, leading to the excretion of GlcNAc conjugates in urine. Screening for bile acids in urine of SLOS patients revealed elevated levels of 3β,7β-dihydroxychol-5-en-24-oic acid conjugated with GlcNAC (elevated by a factor of 10) and in some cases doubly (GlcNAc sulphate double conjugate elevated by a factor of 20) or triply conjugated with sulphuric acid, glycine (GlcNAc, sulphate, glycine triple conjugate elevated by a factor of 10) or taurine (X-XI).

Hedgehog (Hh) signalling is required for embryonic patterning and regeneration of postembryonic tissue and aberrant Hh signalling has been linked to SLOS [Cooper Nat Genetics 2003, Myers Dev Cell 2013]. In fact, many developmental malformations attributed to SLOS occur in tissues where Hh signalling is required for development [Koide Dev 2006]. DHCR7, the defective enzyme in SLOS, has been implicated to function as a positive regulator of Hh signalling, and the cause of some of the developmental abnormalities seen in SLOS have been attributed to cholesterol deficiency interfering with normal Hh signalling [Cooper Nat Gen 2003, Blassberg HMG 2016]. Alternatively, Koide et al [Dev 2006] have suggested that DHCR7 functions as a negative regulator of Hh signalling and its inhibitory effect is at the level, or downstream, of the oncoprotein Smoothened (Smo). Both of these proposals can be accommodated by the model suggested by Myers *et al* where oxysterols and cholesterol bind to and modulate Smo at different structural regions [Dev Cell 2013]. Smo is a seven-transmembrane protein with extended extracellular and cytoplasmic termini. Hh pathway activation is initiated by binding of cholesterol-modified Hh protein to its receptor Patched 1 (Ptch1) which releases inhibition of Smo and triggers transcription of Hh target genes via Gli transcription factors [Lum Science 2004, Rohatgi Science 2007]. Myers *et al* have shown that an extracellular cysteine-rich domain (CRD) is the site for oxysterol binding to Smo and suggested that oxysterols may stabilise an active Smo conformation induced by loss of Ptch1 mediated repression [Dev Cell 346]. 20S-Hydroxycholesterol (20S-HC) has been shown to activate Smo in vitro [Corcoran PNAS 2006, Nachtergaele Nat Chem Bio 2012] but its presence *in vivo* is under debate [Lin JSBMB 2003, Roberg-Larsen JCA 2012]. Two other oxysterols identified herein, 25H,7O-C (XXII) and 26H,7O-C (XX), are also activators of Smo [Myers Dev Cell 346]. 26H,7O-C (XX) has been previously identified in extracts of retinal pigment epithelial cells, and have been shown to be generated from 7-OC (VIII) by CYP27A1 [Heo JLR 2011] (Figure 2). As 7-OC is derived from 7-DHC by CYP7A1 oxidation, the identification of 25H,7O-C (XXII) and 26H,7O-C (XX) in SLOS plasma lends weight to the hypothesis of Koide et al that DHCR7, which reduces the pool of 7-DHC substrate by metabolism to cholesterol functions as an inhibitor of Hh signalling at the level of Smo [Koide Dev 2006]. Although the inventors did not detect 25H,7O-C or 26H,7O-C in plasma from control patients the presence of down-stream metabolites in plasma indicates that the pathway involving their formation is active in man. Like 26H,7O-C (XX), 3βH,7O-CA (VI) has been identified in retinal pigment epithelial cells, derived by CYP27A1 oxidation of 7-OC [Heo JLR 2011].

A method of modulating Smoothened (Smo) receptor activity comprising administering to a patient in need thereof an amount of Compound VI or Compound XXI, or pharmaceutically acceptable salt thereof is provided for the purposes of information.

A method of modulating (preferably inhibiting) Hedgehog signalling (Hh) comprising administering to a patient in need thereof an amount of Compound VI or Compound XXI, or pharmaceutically acceptable salt thereof is provided for the purposes of information.

A method of treating cancer comprising administering to a patient in need thereof an amount of Compound VI, or pharmaceutically acceptable salt thereof is provided for the purposes of information on. Preferably, the cancer is selected from the group consisting of an adenocarcinoma of the pancreas, prostate, breast, stomach, esophagus or biliary tract; a medulloblastoma or glioma; a small-cell lung cancer; a basal cell carcinoma; a rhabdomyosarcoma; a urothelial carcinoma; a squamous cell carcinoma of the oral cavity; and a hepatocellular carcinoma.

A method of treating a wound comprising administering to a patient in need thereof an amount of Compound VI, or pharmaceutically acceptable salt thereof is provided for the purposes of information.

Acomposition comprising a pharmaceutically acceptable carrier, excipient or diluent and Compound VI, or pharmaceutically acceptable salt thereof is provided for the purposes of information.

### Detailed Description of the Invention

The invention will now be particularly described by way of example with reference to the accompanying diagrammatic drawings in which:

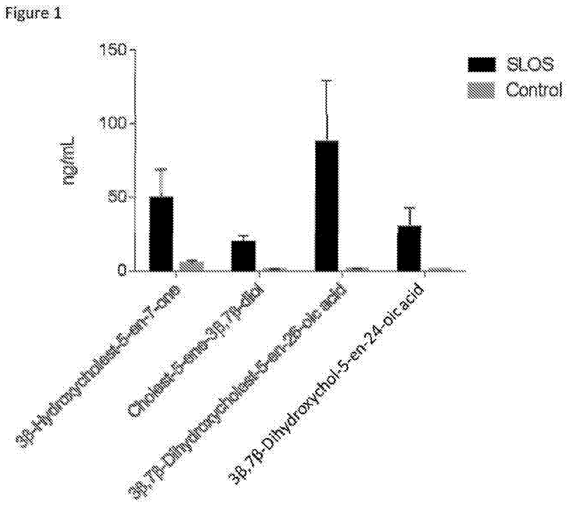

**Figure 1** is a graph showing the concentration of the compounds of formula (VIII)(3β-Hydroxycholest-5-en-7-one or 7-oxocholesterol), compound (VII) (cholest-5-ene-3β,7β-diol or 7β-hydroxycholesterol), compound (IV) (3β,7β-dihydroxycholest-5-en-26-oic acid) and compound (II) (3β,7β-dihydroxychol-5-en-24-oic acid) in plasma from 9 SLOS patients and 50 controls from reference (Theofilopoulos et al JCI 2014). In most control samples the concentration of 3β,7β-dihydroxychol-5-en-24-oic acid was at or below the limit of quantification of 1 ng/mL.
**Figure 2** illustrates the novel bile acid biosynthesis starting with 7-DHC and ending with GlcNAC conjugates of 3β,7β-dihydroxychol-5-en-24-oic acid. The metabolites of elevated abundance found in plasma from SLOS patients are indicated by an upward pointing arrow. Metabolites of elevated abundance found in SLOS urine, and also indicated by an upwards pointing arrow, but are shown in the dashed-box.

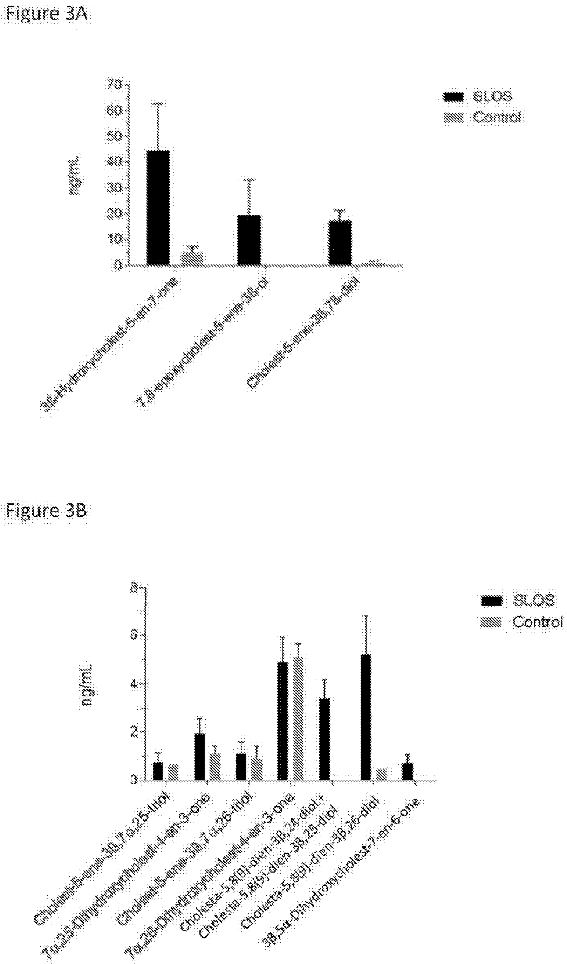

**Figure 3** is a series of graphs showing the concentrations of further compounds that may be used as supplementary diagnostic markers in accordance with an embodiment of the invention, where (A) shows levels of oxysterols enzymatically derived from 7-DHC via oxidation of C-7 and (B) shows levels of dihydroxycholesterols, dihydroxycholestenones and isomers of dihydroxy-8-DHC.

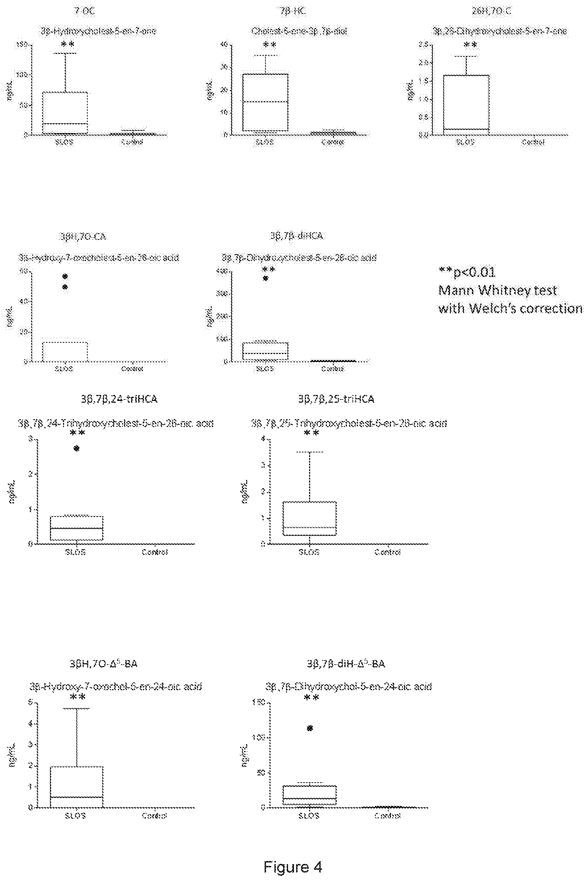

**Figure 4** is a series of graphs illustrating the concentration of 7-OC (VIII), 7β-HC (VII), 26H,7O-C (XX), 3βH,7O-CA (VI), 3β,7β-diHCA (IV), 3β,7β,24-triHCA (III), 3β,7β,25-triHCA (V), 3βH,7O-Δ⁵-BA (XXI) and 3β,7β-diH-Δ⁵-BA (II) in plasma from 10 SLOS patients and 24 controls determined by LC-MS exploiting charge-tagging utilising the GP reagent [Griffiths FRBM 2013, Crick Clin Chem 2015]. The bottom and top of the box are the first and third quartiles, and the band inside the box represents the median. The whiskers extend to the most extreme data points which are no more than 1.5 times the range between first and third quartile distant from the box. Points beyond that are plotted individually. Non-parametric Mann-Whitney test was used for pair-wise comparison for non-normally distributed data. *, P<0.05; **, P<0.01.

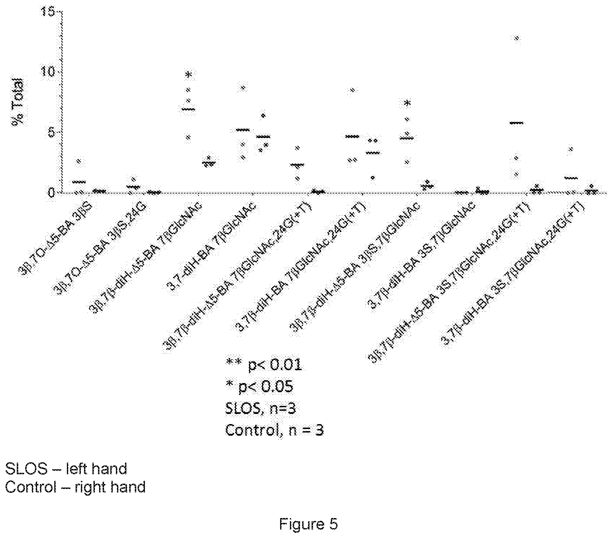

**Figure 5** is a graph showing proportions (mole %) of bile acids with 7-oxo or 7β-hydroxy group conjugated with GlcNAc in urine from 3 SLOS patients and 3 controls determined by LC-MS. Total bile acids include mono-, di- and tri-hydroxylated cholanic acids and their single and doubly unsaturated equivalents singly, doubly or triply conjugated with sulfuric acid, GlcNAc and glycine or taurine. Control data is given on the right hand of each column, SLOS data on the left.

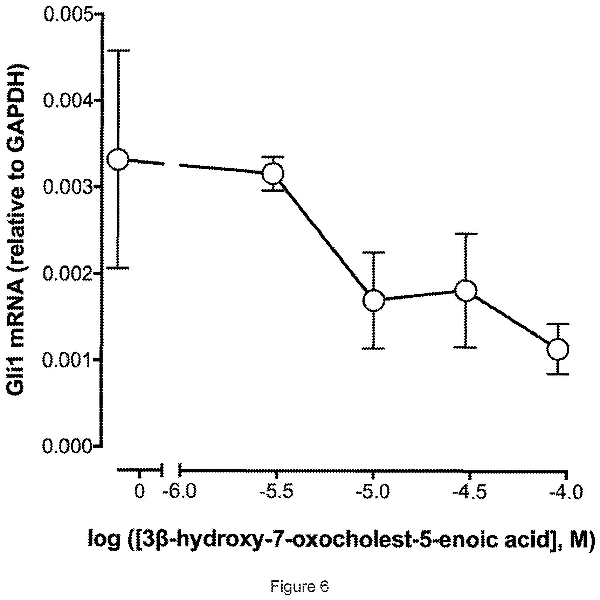

**Figure 6** is a graph showing mRNA levels of Gli1 in H/3T3 cells at different concentrations of 3β-Hydroxy-7-oxocholest-5-enoic acid (Compound XXI).

### Example 1

### Extraction of Sterols and Oxysterols (II-IX, XIII-XXIII) from Plasma

The applicants investigated the possibility that patients suffering from SLOS may use 7-DHC as a starting point for bile acid biosynthesis rather than cholesterol. Liquid chromatography (LC) - mass spectrometry (MS) was used to determine the nature of bile acid intermediates found in plasma from patients suffering from SLOS as well as from healthy individuals as controls. Specifically , compounds (II) - (IX), and (XX) to (XXIII) were identified by a process involving Girard P derivatisation and LC-MS as described by Crick PJ et al., J. Clin Chem. 2015 Feb;61(2):400-11. Compounds (I) and (X) - (XI) were identified in urine by an LC-MS process as described by Griffiths WJ, et al. Mass Spectrometry Handbook, Ed Mike S Lee, 2012 John Wiley & Sons, 2012 p.297 - 337 and elucidated further below.

Plasma (100 µL) was added dropwise to a solution of absolute ethanol (1.05 mL) containing 24R/S-[25,26,26,26,27,27,27-²H₇]hydroxycholesterol ([²H₇]24-OHC) and 22R-[25,26,26,26,27,27,27-²H₇]hydroxycholest-4-en-3-one ([²H₇]22R-OHCO]) (20 ng of each in 1.05 mL of absolute ethanol) in an ultrasonic bath. After 5 min the solution was diluted to 70% ethanol by addition of 0.35 mL of water, ultrasonicated for a further 5 min and centrifuged at 14,000 x g at 4°C for 30 min. The supernatant was loaded onto a 200 mg Certified Sep-Pak C₁₈ cartridge (pre-conditioned with 4 mL of absolute ethanol followed by 6 mL 70% ethanol) and allowed to flow at ∼0.25 mL/min. The flow-through was combined with a column wash of 70% ethanol (5.5 mL) to give SPE1-Fr1 containing the oxysterols. A second fraction (SPE1-Fr2) was collected by eluting with a further 4 mL of 70% ethanol before elution 5 of cholesterol, 7-dehydrocholesterol and similarly hydrophobic sterols using 2 mL of absolute ethanol (SPE1-Fr3). Each fraction was divided into two portions (A) and (B) and concentrated under reduced pressure using a vacuum concentrator (ScanLaf, Denmark).

### Charge Tagging of Sterols and Oxysterols from Plasma

The sterol and oxysterol fractions (A) from above were re-constituted in 100 µL of propan-2-ol then treated with KH₂PO₄ buffer (1 mL 50mM, pH 7) containing 3 µL of cholesterol oxidase (2 mg/mL in H₂O, 44 units/mg protein). The reaction mixture was incubated at 37 °C for 1 hr then quenched with 2.0 mL of methanol. Glacial acetic acid (150 µL) was added followed by Girard P (GP) reagent (190 mg bromide salt or 150 mg chloride salt, 0.80 mmol). The mixture was vortexed then incubated at room temperature overnight in the dark. To remove excess reagent from the reaction mixture a recycling method was used. A 200 mg Certified Sep-Pak C₁₈ cartridge was preconditioned with methanol (6 mL), 10% methanol (6 mL) and finally 70% methanol (4 mL). The derivatization mixture from above (3.25 mL in ∼70% organic) was applied to the column and allowed to flow through at ∼0.25 mL/min. The column was washed with 70% methanol (1 mL) followed by 35% methanol (1 mL) and the combined eluent diluted with water (4 mL) to give a solution in 9 mL of 35% methanol. This solution was applied to the column, collected, and combined with a column wash of 17.5% methanol (1 mL). Water (9 mL) was added to give a solution in 19 mL of 17.5% methanol which was again applied to the column. The flow-through was discarded and the column washed with 10% methanol (6 mL). Derivatized sterols/oxysterols were then eluted from the column with methanol (3 x 1 mL, SPE2-Fr1, Fr2, Fr3) followed by absolute ethanol (1 mL, SPE2-Fr4). Cholesterol and 7-dehydrocholesterol were found to be almost exclusively present in SPE2-Fr3 while oxysterols elute in SPE2-Fr1 and Fr2. The fractions (B) were treated in an identical fashion to the (A) fractions but in the absence of cholesterol oxidase. This allows differentiation of sterols oxidised to contain an oxo group from those naturally possessing one. In later studies the 200 mg Certified Sep-Pak C18 cartridge has been replaced by an Oasis HLB 60-mg column [Crick An Bio Chem 2015].

### LC-MS(MS°) on 5 the LTQ-Orbitrap

To analyse GP-tagged oxysterols, SPE2-Fr1 and -Fr2 were combined and diluted to give a final solution of 60% methanol. For each experiment, 20 µL was injected onto the LC column and MS, MS² and MS³ spectra recorded as described below. For the analysis of the more non-polar sterols SPE2-FR1, -Fr2 and -Fr3 were combined prior to dilution to 60% methanol.

LC was performed on a Ultimate 3000 HPLC system (Dionex, Surrey, UK) using a Hypersil GOLD revered phase column (1.9 µm particle size, 50 x 2.1 mm, Thermo Fisher). Mobile phase A consisted of 33.3% methanol, 16.7% acetonitrile and 0.1% formic acid. Mobile phase B consisted of 63.3% methanol, 31.7% acetonitrile and 0.1% formic acid. The chromatographic run started at 20% B for 1 min before increasing the proportion of B to 80% over 7 minutes and maintaining this for a further 5 min. The proportion of B was returned to 20% over 6 s and re-equilibration was for 3 min, 54 s to give a total run time of 17 min. The flow rate was 200 µL/min and the eluent was directed to the atmospheric pressure ionization (API) source of an LTQ-Orbitrap. The Orbitrap was calibrated externally before each analytical session and the mass accuracy was better than 5 ppm.

The method consisted of a Fourier Transform (FT)-MS scan in the Orbitrap at 30,000 resolution (full width at half-maximum height; FWHM), simultaneous to which sequential MS² or MS³ scans were carried out in the linear ion trap (LIT) with normalised collision energies of for MS² and for MS³ (instrument settings).

Elevated levels of the 7-DHC metabolites, 7-oxocholesterol (Compound VIII), 7β-hydroxycholesterol (Compound VII), 3β, 7β-dihydroxycholest-5-en-26-oic (Compound IV) and 3β, 7β-dihydroxychol-5-en-24-oic (Compound II) acids in SLOS plasma were detected (Figure 1, Figure 4). Also, elevated levels of 3β-hydroxy-7-oxocholest-5-en-26-oic acid (Compound VI), 3β,26-dihydroxycholest-5-en-7-one (26-Hydroxy-7-oxocholesterol) (Compound XX), 3β,7β,24-trihydroxycholest-5-en-26-oic acid (Compound III), 3β,7β,25-trihydroxycholest-5-en-26-oic acid (Compound V) and 3β-hydroxy-7-oxochol-5-en-24-oic acid (Compound XXI) were detected (Figure 4).

In patient samples where the 7-DHC to cholesterol ratio is high, 3β-hydroxy-7-oxocholest-5-en-26-oic acid (Compound VI) was also observed. Low levels of metabolites with retention time and fragmentation patterns consistent with 3β, 7β, 24-trihydroxycholest-5-en-26-oic (Compound III) and 3β, 7β,25-trihydroxycholest-5-en-26-oic structures (Compound V) were also presumptively identified in these patient samples by comparison to the 7α-epimers which were available as authentic standards.

Sterols with a 3β, 7β-dihydroxy-5-ene function are not substrates for HSD3B7, the oxidoreductase required to initiate A/B ring transformation to the 3α-hydroxy-5α-hydrogen configuration found in primary bile acids [Russell ARB 2003], so the 3β, 7β-dihydroxy-5-ene structure is maintained in the products of this bile acid biosynthesis pathway.

### Example 2

### Extraction and Analysis of Bile Acids (I, X, XI) from Urine

Sterols possessing a 7β-hydroxy group are known to be conjugated with N-acetylglucosamine (GlcNAc) and excreted in urine, and so the applicants investigated the urine of SLOS patients for GlcNAc conjugated bile acids using LC-MS methods.

Working solutions of [2,2,4,4-²H₄]cholic acid (20 ng/µL), [2,2,4,4-²H₄]glycochenodeoxycholic acid (20 ng/µL) and [2,2,4,4-²H₄]taurochenodeoxycholic (20 ng/µL) were prepared in absolute ethanol. 2 µL (40 ng) of each working solution was added to 994 µL of water in a 2 mL Eppendorf tube.

Urine (100 µL, pH 6 - 7) was added drop-wise to the 1 mL of water containing deuterated standards (above). After 10 min ultrasonication the solution was centrifuged at 14,000 rpm, 4°C for 30 min and the supernatant 5 retained. An Oasis HLB (60 mg, Waters) column was washed with absolute ethanol (4 mL), methanol (4 mL) and conditioned with water (4 mL). The supernatant from above was loaded onto the column and allowed to flow at 0.25 mL/min. After a 3 mL wash with water bile acids were eluted in 4 x 1 mL of methanol. The first two 1 mL fractions were combined, diluted to 60% methanol and analysed by LC-MS(MS)ⁿ in an identical fashion to derivatised oxysterols as described in Example 1 with the exception that bile acid urine analysis was performed in the negative ion mode.

It was found that, in urine from SLOS patients, there was elevated levels of 3β, 7β-dihydroxychol-5-en-24-oic conjugated with GlcNAc (compound of formula (I)) presumably at position 7β, and also the double conjugate as the 3-sulphate (Figure 5).

### Example 3

### Identification of Additional Sterols and Oxysterols in Plasma

Historical residual clinical plasma samples from SLOS patients were analysed along with samples from newly diagnosed patients and a range of healthy controls.

Sterols and oxysterols were analysed by LC-ESI-MSⁿ using a chargetagging approach (enzyme-assisted derivatisation for sterolanalysis, EADSA) as described in Example 1 above. In brief, nonpolar sterols including cholesterol, 7-DHC and 8-DHC were separated from more-polar oxysterols by reversed-phase solid phase extraction (RP-SPE). The separated fractions were individually treated with cholesterol oxidase to convert 3β-hydroxy-5-ene and 3β-hydroxy-5,7(or 8)-diene to their 3-oxo-4-ene and 3-oxo-4,7(or 8)-diene equivalents, then derivatised with Girard P (GP) reagent to add a charged quaternary nitrogen group to the analytes which greatly improve their LC-ESI-MS and MSⁿ response. When fragmented by MS² GP-tagged analytes give an intense 5 [M-Py]⁺ ion, corresponding to the loss of the pyridine (Py) ring, which can be fragmented further by MS³ to give a structurally informative pattern. Some sterols and oxysterols naturally contain an oxo group and can be differentiated from those oxidised to contain one by omitting the cholesterol oxidase enzyme from the sample work-up procedure.

Representative results from these studies are illustrated in Figure 3. These show that in SLOS patients, compounds of formulae (VII) (VIII), (XVIII), (XIV), (XV), (XVI) and (XIX) are significantly elevated in plasma as compared to those of the normal control samples and that thus these compounds may also give rise to a diagnostic application.

### Example 4

### Hedgehog signalling assays using quantitative RT-PCR

NIH/3T3 cells were grown to confluency in Dulbecco's Modified Eagle's Medium (DMEM) containing 10% Fetal Bovine Serum (FBS, Optima Grade, Atlanta Biologicals). Confluent cells were exchanged into 0.5% FBS DMEM for 24 hours to allow ciliogenesis prior to treatment with sterols in DMEM containing 0.5% FBS for ∼16 hours. SHH protein carrying a C-terminal hexa-histidine tag was expressed in bacteria and purified as described previously [Bishop Nat Struct Mol Biol 2009]. The mRNA levels of Gli1, a direct Hh target gene commonly used as a metric for signalling strength, were measured using the Power SYBR Green Cells-To-CT kit (Thermo Fisher Scientific). The primers used are Gli1 (forward primer: 5'-ccaagccaactttatgtcaggg-3' and reverse primer: 5'-agcccgcttctttgttaatttga-3'), Gapdh (forward primer: 5'-agtggcaaagtggagatt-3' and reverse primer: 5'-gtggagtcatactggaaca-3'). Transcript levels relative to Gapdh were calculated using the Δelta-Ct method. Each qRT-PCR experiment, which was repeated twice, included two biological replicates, each with two technical replicates.

Figure 6 shows results using compound 3β-Hydroxy-7-oxocholest-5-enoic acid (XXI), indicating that this compound is an inhibitor of Hedgehog signalling.

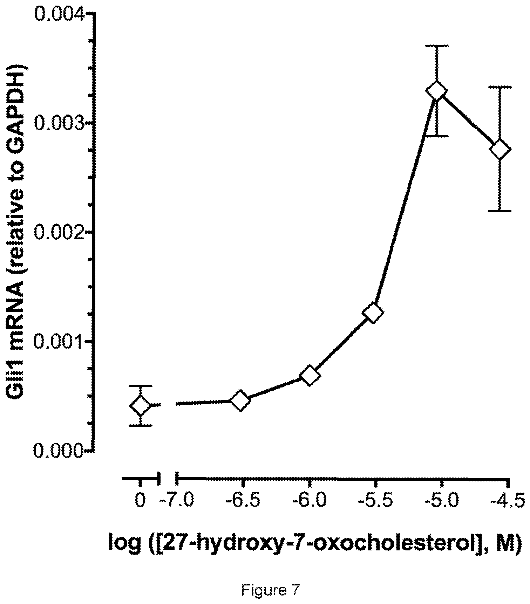

Figure 7 shows the results using 27-hydroxy-7-oxocholesterol (compound XX, alternatively known as 26-hydroxy-7-oxocholesterol), indicating that this compound is an activator of Hedgehog signalling.

### References

Defects in bile acid biosynthesis--diagnosis and treatment. Setchell KD, Heubi JE. J Pediatr Gastroenterol Nutr. 2006 Jul;43 Suppl 1:S17-22.

Cerebrotendinous xanthomatosis: an inborn error in bile acid synthesis with defined mutations but still a challenge. Björkhem I, Hansson M. Biochem Biophys Res Commun. 2010 May 21;396(1):46-9.

Role of a disordered steroid metabolome in the elucidation of sterol and steroid biosynthesis. Shackleton CH. Lipids. 201230 Jan;47(1):1-12.

Identification of unusual 7-oxygenated bile acid sulfates in a patient with Niemann-Pick disease, type C. Alvelius G, Hjalmarson O, Griffiths WJ, Björkhem I, Sjövall J. J Lipid Res. 2001 Oct;42(10):1571-7.

Cholestenoic acids regulate motor neuron survival via liver X receptors. Theofilopoulos S, Griffiths WJ, 5 Crick PJ, Yang S, Meljon A, Ogundare M, Kitambi SS, Lockhart A, Tuschl K, Clayton PT, Morris AA, Martinez A, Reddy MA, Martinuzzi A, Bassi MT, Honda A, Mizuochi T, Kimura A, Nittono H, De Michele G, Carbone R, Criscuolo C, Yau JL, Seckl JR, Schüle R, Schöls L, Sailer AW, Kuhle J, Fraidakis MJ, Gustafsson JÅ, Steffensen KR, Björkhem I, Ernfors P, Sjövall J, Arenas E, Wang Y. J Clin Invest. 2014 Nov;124(11):4829-42.

The enzymes, regulation, and genetics of bile acid synthesis. Russell DW. Annu Rev Biochem. 2003;72:137-74.

Analytical strategies for characterization of oxysterol lipidomes: liver X receptor ligands in plasma. Griffiths WJ, Crick PJ, Wang Y, Ogundare M, Tuschl K, Morris AA, Bigger BW, Clayton PT, Wang Y. Free Radic Biol Med. 2013 Jun;59:69-84.

| **PLASMA** | **CODE** | **SLOS** | **SLOS** | **NIST SRM(1)** | | **From Griffiths (2)** | **From Theofilopoulos (3)** |
|---|---|---|---|---|---|---|---|
| **Sterol Systematic Name (Common name)** | | **Mean** | **SEM** | **Mean** | **SD** | **Mean ±SEM** | **Mean±SEM** |
| 3β,7β-Dihydroxychol-5-en-24-oic acid | (II) | 27.22 | 11.71 | 1.49 | | NM | NM |
| Cholesta-5,8-diene-3β,24(or25)-diol | (XIV+XV) | 3.42 | 0.79 | ND | | NM | NM |
| Cholesta-5,8-diene-3β,26-diol | (XVI) | 5.21 | 1.61 | 0.05 | | NM | NM |
| 7,8-Epoxycholest-5-en-3β-ol | (XVIII) | 19.51 | 13.59 | ND | | NM | NM |
| Cholest-5-ene-3β,7β-diol (7β-Hydroxycholesterol) | (VII) | 17.10 | 4.40 | 0.48 | 0.28 | 0.00±0.32 | 1.02±0.58 |
| 3β-Hydroxycholest-5-en-7-one (7-Oxocholesterol) | (VIII) | 44.55 | 17.82 | 0.59 | 0.33 | 3.77±1.29 | 4.98±2.25 |
| 3β,5α-Dihydroxycholest-7-en-6-one | (XIX) | 0.72 | 0.35 | ND | | NM | NM |
| 3β,7β-Dihydroxycholest-5-en-26-oic acid | (IV) | 78.86 | 38.16 | 2.74 | 0.18 | 5.36±0.80 | 1.67±0.32 |
| 3β-Hydroxy-7-oxocholest-5-en-26-oic acid | (VI) | 11.87 | 7.86 | 0.04 | | NM | NM |
| 3β,7β,24-Trihydroxycholest-5-en-26-oic acid | (III) | 0.50 | | ND | | NM | NM |
| 3β,7β,25-Trihydroxycholest-5-en-26-oic acid | (V) | 0.63 | | ND | | NM | NM |
| | | | | | | | |
| ND not detected. NM not measured | | | | | | | |
| (1) NIST standard reference material 1950. Pooled sample, representative of the USA population | | | | | | | |
| (2) Analytical strategies for characterization of oxysterol lipidomes: liver X receptor ligands in plasma. Griffiths WJ, Crick PJ, Wang Y, Ogundare M, Tuschl K, Morris AA, Bigger BW, Clayton PT, Wang Y. Free Radic Biol Med. 2013 Jun;59:69-84. | | | | | | | |
| (3) Cholestenoic acids regulate motor neuron survival via liver X receptors. Theofilopoulos S, et al. J Clin Invest. 2014 Nov;124(11):4829-42 | | | | | | | |

## Claims

1. A method for diagnosing Smith-Lemli-Opitz syndrome (SLOS) comprising detecting levels of the compound of formula (I) or a derivative thereof in a urine sample from a subject suspected of or suffering from SLOS, or from a urine sample from an expectant mother 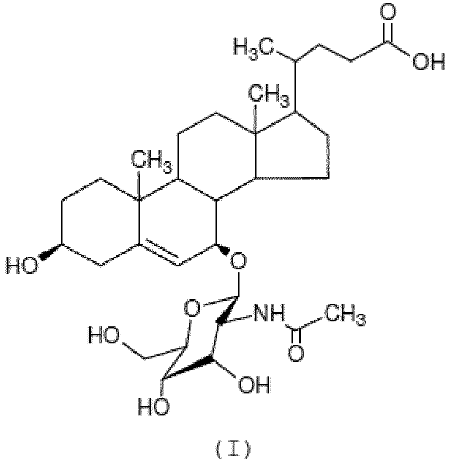 comparing these with the levels found in healthy subject
which are higher than those found in a sample from a subject not suffering from SLOS.

2. A method according to claim 1 wherein the derivative of formula (I) is a compound of formula (X) or (XI) 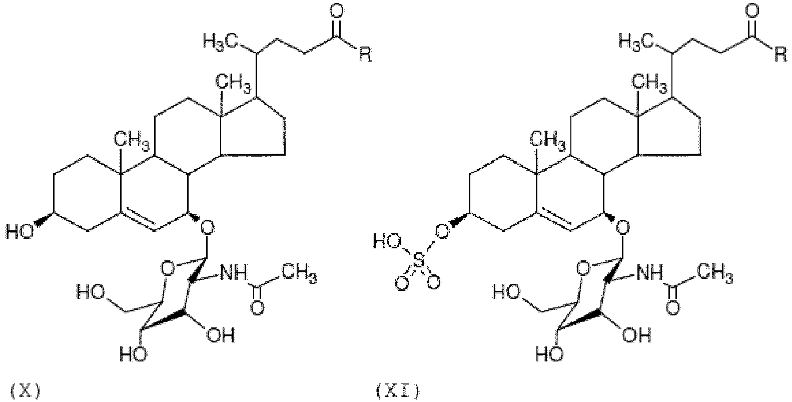 wherein R is a hydroxyl, glycine or taurine group.

3. A method according to any one of the preceding claims which further comprises detecting and/or quantifying a further compound or diagnostic marker.

4. A method according to claim 3 wherein a further compound is 8-dehydocholesterol (8-DHC) of formula (XIII) 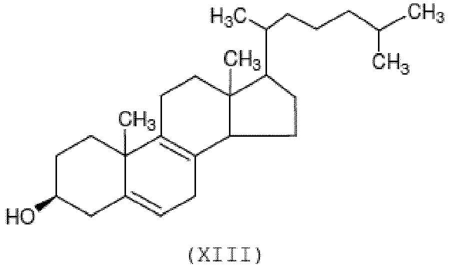 or a metabolite thereof, selected from 24-hydroxy-8-DHC (24-OH-8-DHC) of formula (XIV), 25-OH-8-DHC of formula (XV) and 26-OH-8-DHC of formula (XVI),

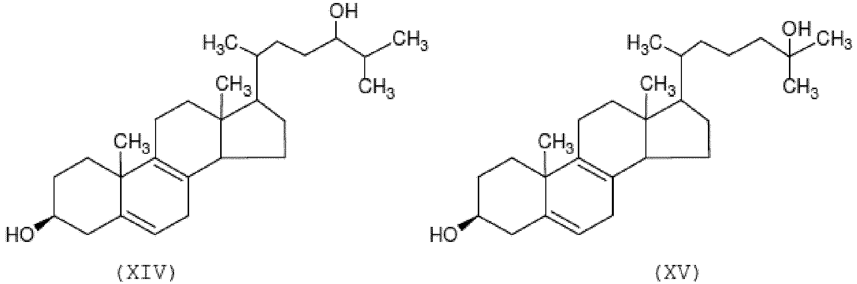

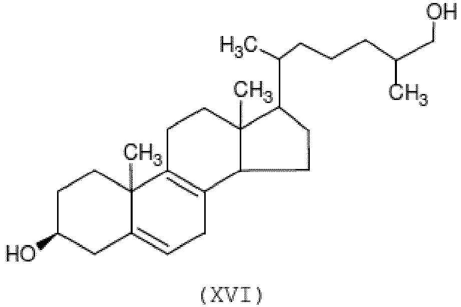

5. A method according to claim 4 wherein a further compound is the compound of formula (XIV) or (XV).

6. A method according to any one of claims 3 to 5 wherein a further compound is 7-DHC of formula (IX) 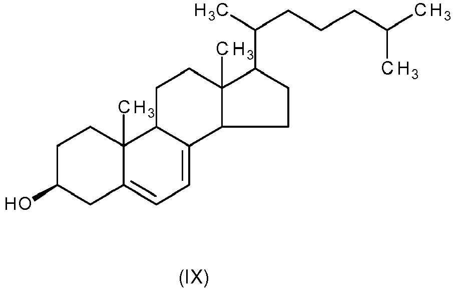 or 7-DHC metabolites.

7. A method according to claim 6 wherein the 7-DHC metabolites are selected from 4-OH-7-DHC of formula (XVII), 7-oxocholesterol (Compound VIII), 7α,8α-epoxycholesterol (XVIII) and 3β,5α-dihydroxycholest-7-en-6-one (Compound XIX)

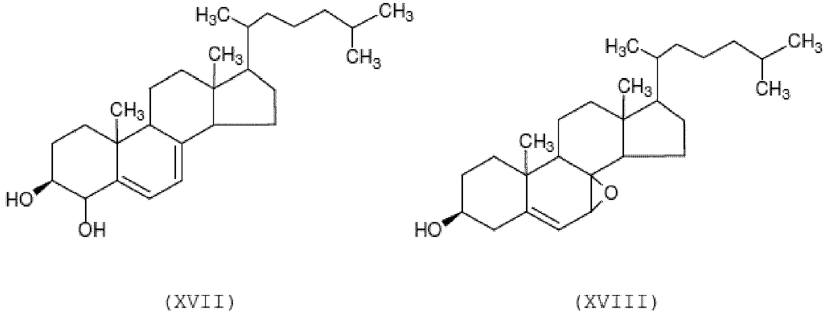

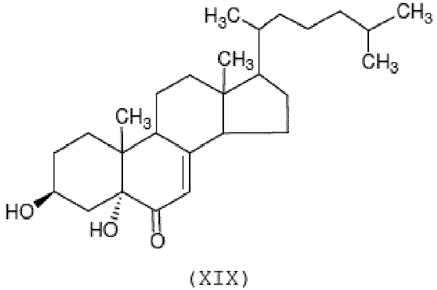

8. A method according to any one of the preceding claims wherein the compounds detected are detected and/or quantified using liquid chromatography or mass spectrometry or a combination thereof.

9. A method according to claim 8 wherein a compound is derivatised by reaction with a conjugation agent to facilitate detection.

10. A method according to claim 9 wherein the conjugation agent is a Girard reagent.

## Patentansprüche

1. Verfahren zur Diagnose des Smith-Lemli-Opitz-Syndroms (SLOS), das Folgendes umfasst: Nachweisen von Höhen der Verbindung der Formel (I) oder eines Derivats davon in einer Urinprobe von einem Subjekt, bei dem SLOS vermutet wird oder das daran leidet, oder von einer Urinprobe von einer werdenden Mutter: 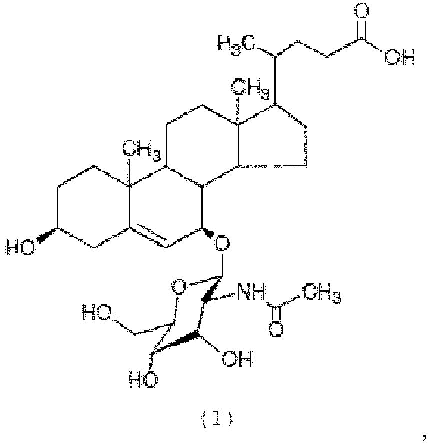 Vergleichen dieser mit den Höhen, die bei einem gesunden Subjekt ermittelt werden, die höher sind als die, die in einer Probe von einem Subjekt ermittelt werden, das nicht an SLOS leidet.

2. Verfahren nach Anspruch 1, wobei das Derivat der Formel (I) eine Verbindung der Formel (X) oder (XI) ist: 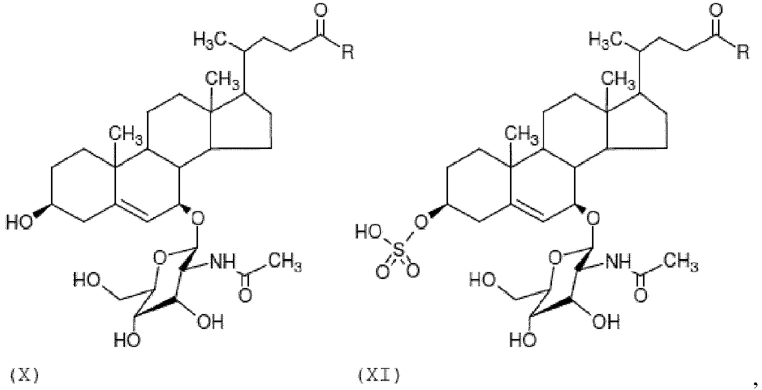 wobei R eine Hydroxyl-, Glycin- oder Tauringruppe ist.

3. Verfahren nach einem der vorstehenden Ansprüche, das weiter das Nachweisen und/oder das Quantifizieren einer weiteren Verbindung oder eines diagnostischen Markers umfasst.

4. Verfahren nach Anspruch 3, wobei eine weitere Verbindung Folgendes ist: 8-Dehydrocholesterol (8-DHC) der Formel (XIII): 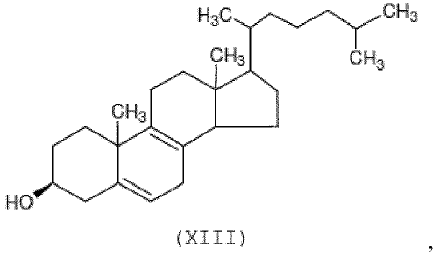 oder ein Metabolit davon, der aus 24-Hydroxy-8-DHC (24-OH-8-DHC) der Formel (XIV), 25-OH-8-DHC der Formel (XV) und 26-OH-8-DHC der Formel (XVI) ausgewählt ist:

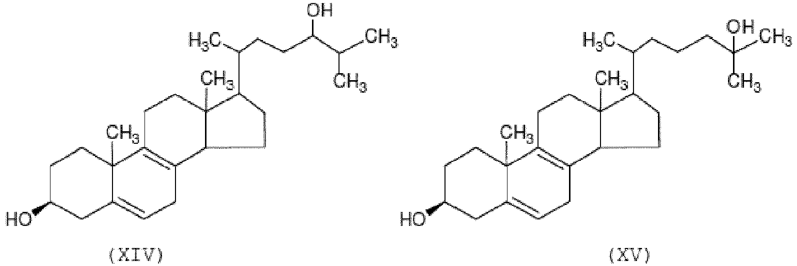

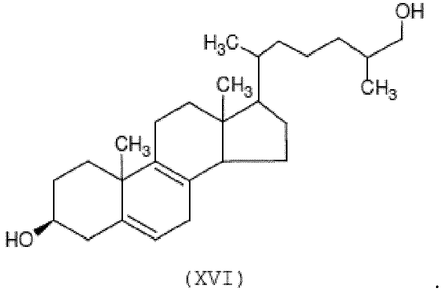

5. Verfahren nach Anspruch 4, wobei eine weitere Verbindung die Verbindung der Formel (XIV) oder (XV) ist.

6. Verfahren nach einem der Ansprüche 3 bis 5, wobei eine weitere Verbindung 7-DHC der Formel (IX): 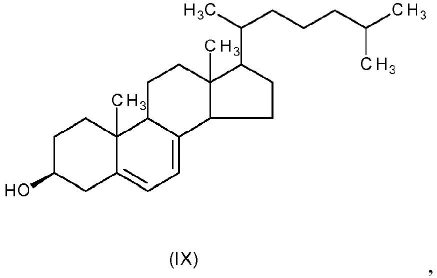 oder 7-DHC-Metaboliten ist.

7. Verfahren nach Anspruch 6, wobei die 7-DHC-Metaboliten aus 4-OH-7-DHC der Formel (XVII), 7-Oxocholesterol (Verbindung VIII), 7a,8a-Epoxycholesterol (XVIII) und 3β,5α-Dihydroxycholest-7-en-6-on (Verbindung XIX) ausgewählt sind:

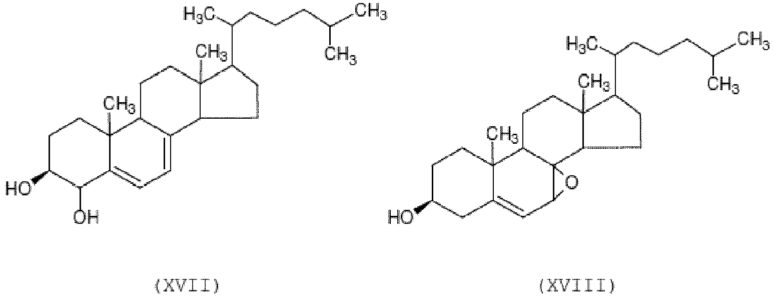

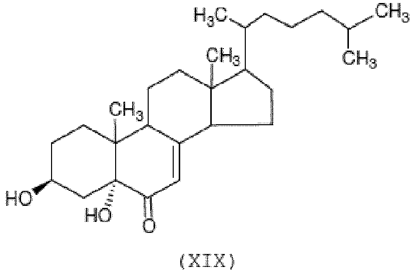

8. Verfahren nach einem der vorstehenden Ansprüche, wobei die nachgewiesenen Verbindungen unter Verwendung von Flüssigchromatographie oder Massenspektrometrie oder einer Kombination davon nachgewiesen und/oder quantifiziert werden.

9. Verfahren nach Anspruch 8, wobei eine Verbindung durch Reaktion mit einem Konjugationsreagenz derivatisiert wird, um den Nachweis zu ermöglichen.

10. Verfahren nach Anspruch 9, wobei das Konjugationsreagenz ein Girard-Reagenz ist.

## Revendications

1. Méthode de diagnostic du syndrome de Smith-Lemli-Opitz (SLOS), comprenant la détection des taux du composé de formule (I) ou d'un dérivé de celui-ci dans un échantillon d'urine issu d'un sujet suspecté de souffrir ou souffrant du SLOS, ou dans un échantillon d'urine issu d'une femme enceinte 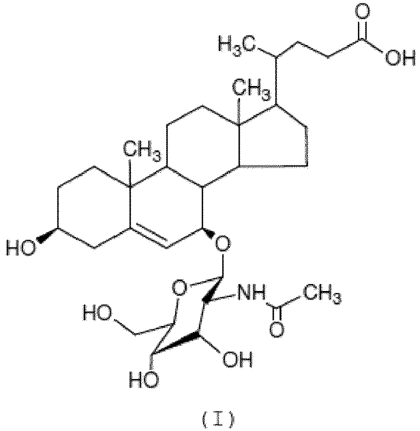 la comparaison de ceux-ci avec les taux rencontrés chez un sujet sain qui sont supérieurs à ceux rencontrés dans un échantillon issu d'un sujet ne souffrant pas du SLOS.

2. Méthode selon la revendication 1, dans laquelle le dérivé de formule (I) est un composé de formule (X) ou (XI) 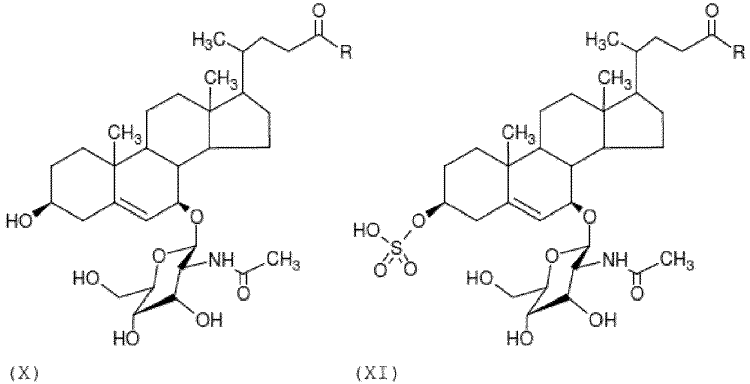 où R est un groupement hydroxyle, glycine ou taurine.

3. Méthode selon l'une quelconque des revendications précédentes, comprenant en outre la détection et/ou la quantification d'un autre composé ou marqueur de diagnostic.

4. Méthode selon la revendication 3, dans laquelle un autre composé est le 8-déshydrocholestérol (8-DHC) de formule (XIII) 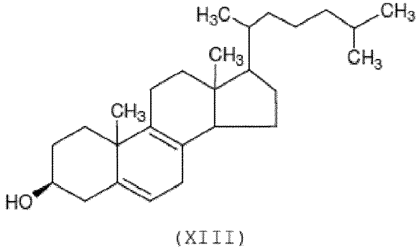 ou un métabolite de celui-ci, choisi parmi le 24-hydroxy-8-DHC (24-OH-8-DHC) de formule (XIV), le 25-OH-8-DHC de formule (XV) et le 26-OH-8-DHC de formule (XVI)

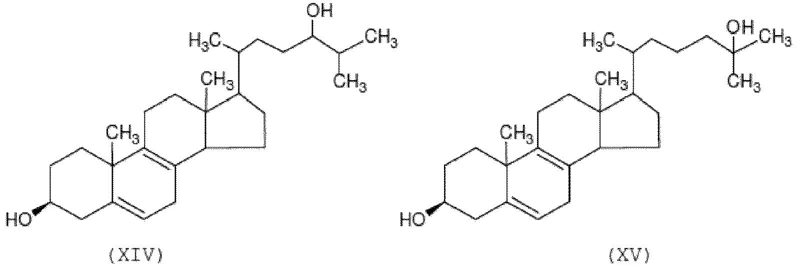

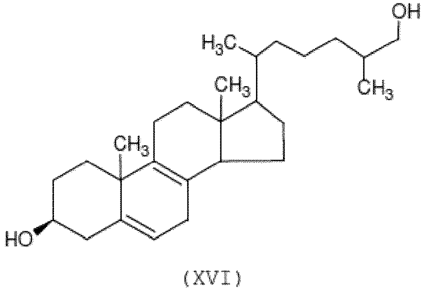

5. Méthode selon la revendication 4, dans laquelle un autre composé est le composé de formule (XIV) ou (XV).

6. Méthode selon l'une quelconque des revendications 3 à 5, dans laquelle un autre composé est le 7-DHC de formule (IX) 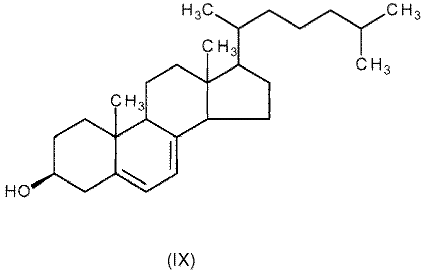 ou des métabolites de 7-DHC.

7. Méthode selon la revendication 6, dans laquelle les métabolites de 7-DHC sont choisis parmi le 4-OH-7-DHC de formule (XVII), le 7-oxocholestérol (Composé VIII), le 7α-8α-époxycholestérol (XVIII) et la 3β,5α-dihydroxycholest-7-én-6-one (Composé XIX)

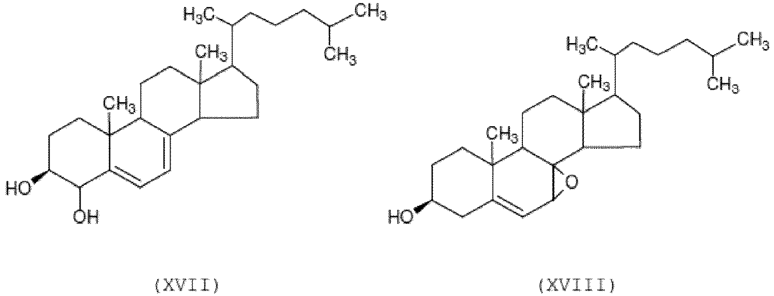

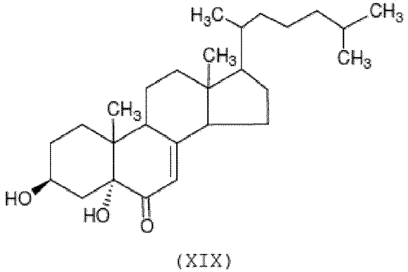

8. Méthode selon l'une quelconque des revendications précédentes, dans laquelle les composés détectés sont détectés et/ou quantifiés par chromatographie liquide ou spectrométrie de masse, ou une combinaison de celles-ci.

9. Méthode selon la revendication 8, dans laquelle un composé est dérivatisé par réaction avec un agent de conjugaison afin de faciliter la détection.

10. Méthode selon la revendication 9, dans laquelle l'agent de conjugaison est un réactif de Girard.
